(19) Europäisches Patentamt
European Patent Office
Office européen des brevets

(11) EP 3 407 029 A1

(12) EUROPEAN PATENT APPLICATION

(43) Date of publication:
28.11.2018 Bulletin 2018/48

(51) Int Cl.:
G01F 1/66 (2006.01)          A61B 5/00 (2006.01)
G01F 1/32 (2006.01)          G01P 5/10 (2006.01)

(21) Application number: 17172532.8

(22) Date of filing: 23.05.2017

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
MA MD

(71) Applicant: Nokia Technologies Oy
02610 Espoo (FI)

(72) Inventors:
• Rajala, Satu
  02680 Espoo (FI)

• Blomqvist, Kim
  02650 Espoo (FI)
• Huttunen, Janne
  02610 Espoo (FI)
• Müller, Kiti
  00430 Helsinki (FI)
• Kärkkäinen, Leo
  00100 Helsinki (FI)

(74) Representative: Potter Clarkson LLP
The Belgrave Centre
Talbot Street
Nottingham NG1 5GG (GB)

(54) **AN APPARATUS, METHOD AND COMPUTER PROGRAM FOR MEASURING A FLOW OF MILK EXPRESSED FROM A MAMMARY GLAND**

(57)    An apparatus comprising:
a nipple chamber having opposing first and second chamber ends, the chamber configured to receive a nipple of a mammary gland though the first chamber end and dimensioned to hold the nipple-end of a received nipple proximal to the opposing second chamber end, the second chamber end being substantially closed with one or more apertures therein to allow milk from the received nipple to flow out of the second chamber end and produce turbulence in the milk flow; and
a microphone located proximal to the nipple chamber and configured to receive an acoustic signal produced by the turbulent milk flow in the nipple chamber.

Figure 2a

## Description

Technical Field

The present disclosure relates to apparatus, methods and computer programs for measuring a flow of milk expressed from a mammary gland.

Background

Breast feeding is known to have numerous benefits for both mother and baby which infant formula lacks.
The listing or discussion of a prior-published document or any background in this specification should not necessarily be taken as an acknowledgement that the document or background is part of the state of the art or is common general knowledge.

Summary

According to a first aspect, there is provided an apparatus comprising:

a nipple chamber having opposing first and second chamber ends, the chamber configured to receive a nipple of a mammary gland though the first chamber end and dimensioned to hold the nipple-end of a received nipple proximal to the opposing second chamber end, the second chamber end being substantially closed with one or more apertures therein to allow milk from the received nipple to flow out of the second chamber end and to produce turbulence in the milk flow; and
a microphone located proximal to the nipple chamber and configured to receive an acoustic signal produced by the turbulent milk flow in the nipple chamber.

It will be appreciated that one or more dimensional properties of the apparatus, as discussed below, can be chosen in various combinations to provide for the required turbulent milk flow in the nipple chamber.
One or more of the walls, or the radial wall, of the nipple chamber may be within a proximity of at least one of 5 mm, 4 mm, 3 mm, 2 mm and 1 mm of the received nipple.
The nipple chamber of the apparatus may be sized to achieve this for a typical range of nipple sizes for a population, e.g. an adult female human population. Separate apparatus could be manufactured for different classes of nipple size -for example, the adult female human population could be classified into 'small, 'medium' and 'large' average nipple sizes, and separate 'small', 'medium' and 'large' apparatus could be manufactured such that one or more of the walls, or the radial wall, of the nipple chamber is within a proximity of at least one of 5 mm, 4 mm, 3 mm, 2 mm and 1 mm of a respective 'small', 'medium' and 'large' received nipple of the average size for the respective class. It will be appreciated that this feature should not be interpreted as limited to 'within a proximity of 5 mm, 4mm, 3 mm, 2mm and 1 mm of the received nipple whilst the apparatus is in use on a mammary gland'. Instead, this feature encompasses an apparatus with a nipple chamber which has these dimensions relative to an average nipple size (e.g. for that class). It will be appreciated that said sizes/classes could also vary according to populations in respective geographic regions (e.g. small in the US could be different to small in the UK).
The distance between the nipple-end and the second chamber end may be less than one or more of 10 mm, 9 mm, 8 mm, 7 mm, 6 mm, 5 mm, 4 mm, 3 mm, 2 mm, 1 mm. As in the previous paragraph, it will be appreciated that the nipple chamber of the apparatus may be sized to achieve this for a typical range of nipple sizes for a population, e.g. an adult female human population, and several apparatus could be manufactured for different classes of nipple size. As above, this feature should not be interpreted as limited to 'the distance between the (received) nipple-end and the second chamber end, whilst the apparatus is in use on a mammary gland'. Instead, this feature encompasses an apparatus with a nipple chamber which has these dimensions relative to an average nipple size.
The nipple chamber may have a radial dimension of 7-25 mm. The nipple chamber may have a longitudinal dimension of 15-50 mm.
The shape of the one or more apertures in the second chamber end may be circular, oval or polygonal.
The size of an appropriate dimension of the one or more apertures in the second chamber end may be in the range of 0.5 mm to 5 mm. This appropriate dimension may be the diameter of a circular aperture, a major or minor diameter of an oval aperture or the side length of a regular polygonal aperture.
The cumulative cross-sectional area of the one or more apertures may be one or more of up to 5 %, 10 %, 15 % and 20 % of the total surface area of the second chamber end of the nipple chamber. Accordingly, the second chamber end may be considered to be "substantially closed" by virtue of this relation between the total surface area of the second chamber end and the cumulative cross-sectional area of the one or more apertures therein.
The apparatus may further comprise one or more structures on an interior wall of the nipple chamber, the one or more structures configured to increase turbulence in the milk flow. The structures may comprise one or more of: protrusions, concavities, hydrophilic regions and hydrophobic regions.
It will be appreciated that the above mentioned dimensional properties of the apparatus can be chosen in various combinations to provide for the required turbulent milk flow in the nipple chamber.
The apparatus may further comprise:

at least one processor; and
at least one memory including computer program code,
the at least one memory and the computer program code configured to, with the at least one processor, cause the apparatus to perform at least the following:

    measure the acoustic signal produced by the turbulent milk flow in the nipple chamber; and
    determine the speed of milk flow through the one or more apertures using the measured acoustic signal.

The apparatus may determine the speed of milk flow through the one or more apertures using the measured acoustic signal by:

    determining, from the acoustic signal, the frequency of vortex shedding in the turbulent milk flow; and
    determining the speed using the determined frequency.

The apparatus may use the determined speed of milk flow to determine a volumetric flow rate of milk flow through the one or more apertures. The apparatus may use the determined speed of milk flow to determine a volume of milk expressed by the mammary gland.

The mammary gland may be a human mammary gland.

The microphone may be one or more of:

    embedded within a wall of the nipple chamber such that the surface roughness of the interior surface of the wall in the region where the microphone is embedded is within 5% of the surface roughness of the interior surface of the wall in the regions where the microphone is not embedded;
    removably located in a microphone cavity in a wall of the nipple chamber, the microphone cavity positioned and dimensioned such that the surface roughness of the interior surface of the wall in the region of the microphone cavity is within 5% of the surface roughness of the interior surface of the wall in the regions away from the microphone cavity;
    located on an exterior or interior wall surface of the nipple chamber; or
    located at an opening of a cavity within a wall of the nipple chamber, wherein the opening is located at a wall surface of the nipple chamber and the cavity is shaped and dimensioned so that it provides an acoustic impedance match with the microphone.

The cavity within a wall of the nipple chamber may comprise a chamber and a conduit, wherein the opening of the cavity at the wall surface of the nipple chamber is an opening of the conduit at the wall surface, and wherein the chamber is shaped and dimensioned so that it provides an acoustic impedance match with the microphone.

According to a second aspect, there is provided an apparatus comprising:

    at least one processor; and
    at least one memory including computer program code,
    the at least one memory and the computer program code configured to, with the at least one processor, cause the apparatus to perform at least the following:

        receive an acoustic signal from a microphone located proximal to the nipple chamber of a second apparatus, wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk from the received nipple to flow out of the second chamber end and to produce turbulence in the milk flow, wherein the received acoustic signal is produced by the turbulent milk flow; and
        determine the speed of milk flow through the one or more apertures using the received acoustic signal.

The microphone may be located proximal to the nipple chamber of the second apparatus by being comprised by the second apparatus. The microphone may be separate from the second apparatus.

The computer program code may be further configured to cause the apparatus to:

    determine, from the acoustic signal, the frequency of vortex shedding in the turbulent milk flow; and
    determine the speed of milk flow through the one or more apertures using the determined frequency.

The computer program code may be further configured to cause the apparatus to determine the speed of milk flow using the determined frequency, the Strouhal number for milk flow through the nipple chamber and a characteristic length of the nipple chamber.

The computer program code may be further configured to cause the apparatus to use the determined speed of milk flow to determine a volumetric flow rate of milk flow through the one or more apertures.

The computer program code may be further configured to cause the apparatus to use the determined speed of milk flow to determine a volume of milk expressed by the mammary gland.

According to a third aspect, there is provided a method comprising:

receiving an acoustic signal from a microphone located proximal to the nipple chamber of an apparatus wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk from the received nipple to flow out of the second chamber end and to produce turbulence in the milk flow, wherein the received acoustic signal is produced by the turbulent milk flow; and

determining the speed of milk flow through the one or more apertures using the received acoustic signal.

According to a fourth aspect, there is provided a computer program configured to cause an apparatus to:

receive an acoustic signal from a microphone located proximal to the nipple chamber of a second apparatus, wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk from the received nipple to flow out of the second chamber end and to produce turbulence in the milk flow, wherein the received acoustic signal is produced by the turbulent milk flow; and determine the speed of milk flow through the one or more apertures using the received acoustic signal.

The steps of any method disclosed herein do not have to be performed in the exact order disclosed, unless explicitly stated or understood by the skilled person.

Corresponding computer programs for implementing one or more steps of the methods disclosed herein are also within the present disclosure and are encompassed by one or more of the described example embodiments.

One or more of the computer programs may, when run on a computer, cause the computer to configure any apparatus to perform any method disclosed herein. One or more of the computer programs may be software implementations, and the computer may be considered as any appropriate hardware, including one or more of a digital signal processor, a microcontroller, Read Only Memory (ROM), Erasable Programmable Read Only Memory (EPROM) and Electronically Erasable Programmable Read Only Memory (EEPROM), as non-limiting examples. The software may be a manufacture or assembly program for manufacturing/assembling one or more of the apparatus disclosed herein.

One or more of the computer programs may be provided on a computer readable medium, which may be a physical computer readable medium such as a disc or a memory device or other non-transient tangible medium, or may be embodied as a transient signal. Such a transient signal may be a network download, including an internet download.

The present disclosure includes one or more corresponding aspects, example embodiments or features in isolation or in various combinations whether or not specifically stated (including claimed) in that combination or in isolation. Corresponding means for performing one or more of the discussed functions are also within the present disclosure.

The above summary is intended to be merely exemplary and non-limiting.

Brief Description of the Figures

A description is now given, by way of example only, with reference to the accompanying drawings, in which:-

Figure 1 shows a diagram of a nipple shield;
Figure 2a shows one example of a first apparatus as described herein;
Figure 2b shows a cross-sectional view of the apparatus shown in Figure 2a;
Figure 3 shows another example of the present apparatus;
Figure 4 shows another example of the present apparatus;
Figure 5 shows another example of the present apparatus;
Figure 6 shows another example of the present apparatus;
Figure 7 shows the creation of turbulence in the milk flow through the nipple chamber;
Figure 8 shows one example of a second apparatus as described herein;
Figure 9 shows one example of a first apparatus connected to a second apparatus as described herein;
Figure 10 shows the main steps of a method of using an apparatus as described herein; and
Figure 11 shows a computer-readable medium comprising a computer program configured to perform, control or enable a method described herein.

Detailed Description

There will now be described apparatus, associated methods and computer programs that may provide an improved way to measure a flow of milk expressed from a mammary gland using a measured acoustic signal produced by turbulent milk flow. In some example embodiments the acoustic signal is produced by vortex shedding in turbulent milk flow.

In fluid dynamics, a turbulent fluid flow is characterized by a non-zero vorticity. Vortices can be understood as regions of fluid in which the flow is rotating around an axis; real-world examples include aeroplane wingtip vortices and smoke rings. Vortex shedding should be understood as an oscillating pattern of turbulent flow - for example, a repeating pattern of swirling vortices (Kármán vortex sheet) or circular vortices - produced by an obstruction or a constriction in a fluid flow. Vortex shedding involves vortices being created at or near the obstruction or constriction and periodically detaching (or equivalently "shedding") from the obstruction or constriction.

This is in contrast to a laminar flow in which fluid flows in parallel layers with no lateral mixing between these layers. The transition between laminar and turbulent flow regimes is characterised by the dimensionless Reynolds number (with turbulent flow occurring for high Reynolds numbers). The Reynolds number gives the ratio between inertial forces and viscous forces for a fluid flow, and can be defined as $Re=UL/v,$ where $U$ is a characteristic flow speed, $L$ is a characteristic linear dimension ("characteristic length") for the flow and $v$ is the kinematic viscosity of the fluid. Characteristic length $L$ is a linear dimension that is selected to represent the scale of a physical system. Examples include the diameter of a pipe through which fluid is flowing and the diameter of a spherical obstruction to fluid flow. Characteristic flow speed $U$ is the flow speed away from any obstructions or boundaries. It will be appreciated that the Reynolds number for a flow is dependent on the particular configuration thorough or in which flow is occurring, by virtue of the characteristic length $L$ that is selected to represent the scale of the physical system. For example, different types of configuration (e.g. flow through a cylindrical pipe or between parallel plates) and different sizes and/or shapes of configuration (e.g. a thin cylindrical pipe, a thick cylindrical pipe and a thin rectangular pipe) can have different Reynolds numbers.

In the present disclosure, discussion of turbulent flows should be understood as a flow that is at least partially turbulent, i.e. a fluid flow which has turbulent character in at least some regions of the fluid but which may have laminar character in other regions.

It should also be understood through the present disclosure that a determined flow speed can be used to determine a volumetric flow rate (= flow speed x cross-sectional area of the one or more apertures) and/or a volume of expressed milk (= flow speed x cross-sectional area of the one or more apertures, integrated with respect to time for the duration of expressing session). One or more apparatus described herein may be configured to determine one or more of volumetric flow rate and volume of expressed milk as well as speed of milk flow.

Throughout the present disclosure, the term "mammary gland" should be understood to mean a complex mammary gland such as a breast (in primates, e.g. humans) or an udder (in ruminants, e.g. cows, goats). The term "nipple" should be understood to mean a mammary papilla, for examples a nipple of a human breast or a teat of a cow's udder. The term "nipple-end" should be understood to mean the part of the nipple that is furthest from the rest of the mammary gland.

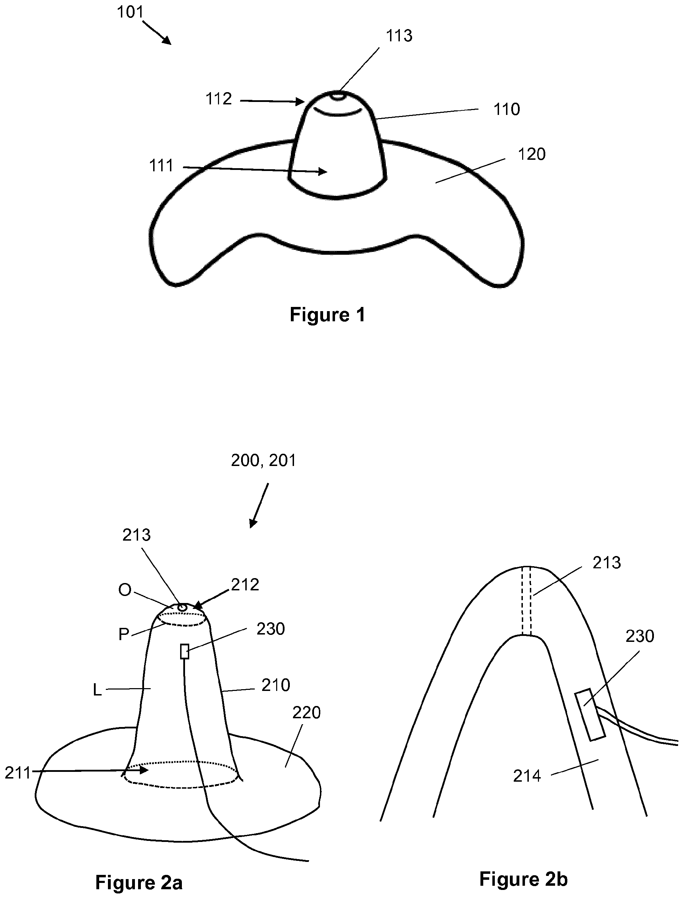

Figure 1 shows a nipple shield 101 comprising a nipple chamber 110 and a base portion 120. The nipple chamber 110 is dimensioned to allow a nipple of a mammary gland to be received therein. The nipple chamber 110 has opposing first 111 and second 112 chamber ends. The first chamber end 111 is open to allow a nipple of a mammary gland (e.g. a human breast) to be received in the nipple chamber 110 through the first chamber end 111. The second chamber end 112 is substantially closed, with one or more apertures 113 therein. When in use, the nipple shield 101 is placed over the nipple and a portion of the mammary gland. The nipple shield 101 may be positioned to form a seal between the nipple shield and the mammary gland. The nipple is held within the nipple chamber 110, and expressed milk flows from the nipple through the nipple chamber 110 and out of the apertures 113 in the second chamber end 112 (e.g. into a baby's mouth).

Figure 2a shows an apparatus 200 according to an example embodiment of the present invention. In this example embodiment, the apparatus 200 is a nipple shield 201 comprising a nipple chamber 210 and a base portion 220. Similarly to Figure 1, nipple chamber 210 has opposing first 211 and second 212 chamber ends, where the first chamber end 211 is open and the second chamber end 212 is substantially closed with at least one aperture 213 therein. The perimeter P of the second chamber end (demarcated with a dotted line) is chosen such that it encloses an area O which is opposing to the open first chamber end 211 and does not include areas L which are lateral to the first chamber end 211. The apparatus 200 also comprises a microphone 230.

Figure 2b shows a cross-sectional view of the nipple chamber 210 shown in Figure 2a. In Figure 2b, the microphone 230 is embedded in a radial wall 214 of the nipple chamber 210. The microphone 230 is embedded such that the microphone 230 does not substantially protrude into the nipple chamber 210, and this can be parameterized using the surface roughness of the interior surface of the wall 214 in which the microphone is embedded. In some example embodiments, the surface roughness of the interior surface of the wall 214 in the region where the microphone 230 is embedded is within 5% of the surface roughness of the interior surface of the wall 214 in the regions where the microphone 230 is not embedded. Other example embodiments may use different threshold values, for example 2%, 10% or 15%. Embodiments with lower surface roughness may be perceived by a mother to be more comfortable when using the nipple shield. Various parameters can be used to measure roughness, including the mean roughness $Ra$ and the root mean square roughness $Rq$.

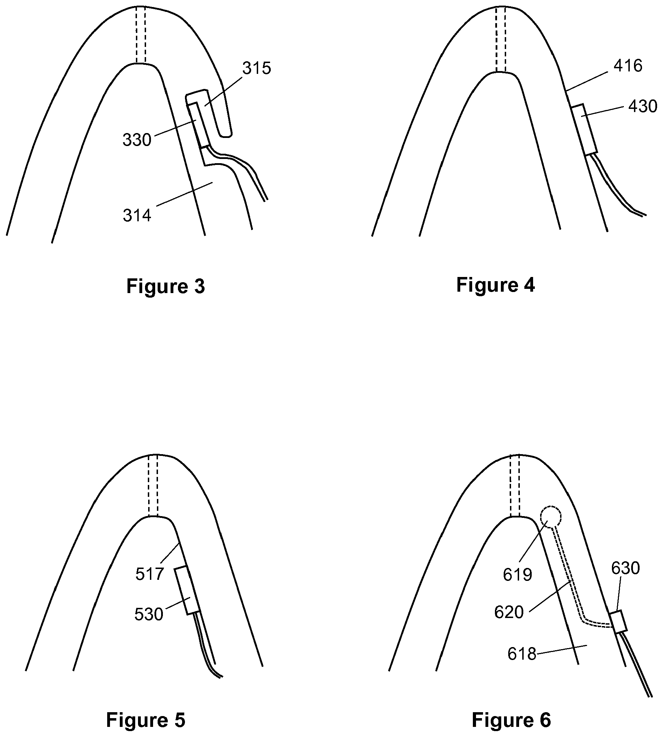

In another example embodiment (shown in Figure 3 in cross-sectional view), the microphone 330 may be placed in a microphone cavity 315 in a wall 314 (e.g. a radial wall) of the nipple chamber. Similarly to the embodiment in Figure 2b, the microphone cavity 315 may be positioned and dimensioned such that the microphone 330 in the microphone cavity 315 does not substantially protrude into the nipple chamber. This can be similarly parameterized using the difference in relative surface roughness between the interior surface of the wall 314 in the region of the microphone cavity 315 and in the regions away from the microphone cavity.

In other example embodiments (shown in Figures 4 and 5 respectively), the microphone 430, 530 may be positioned on an exterior surface 416 or interior surface 517 of the nipple chamber. In another example embodiment (shown in Figure 6), there may be a cavity in a wall 618 of the nipple chamber, the cavity comprising a chamber 619 and a conduit 620. The conduit 620 has an open end (or opening) at a wall face of the nipple chamber, e.g. at an exterior wall surface. The chamber may be shaped and dimensioned so as to provide an acoustic impedance match with a microphone 630 placed at/connected to the open end of the conduit 620. In this way, the chamber acts similarly to the bell in an acoustic stethoscope to transfer acoustic energy from the source of sound to the microphone.

The positioning of the microphone (e.g. in any of the embodiments of Figures 2b-6) may be chosen such that the microphone is sufficiently close to the milk flow to receive an acoustic signal of sufficient strength, taking into account the sensitivity of the microphone. The positioning of the microphone may also be chosen based on ease of manufacture, ease of use for a mother, comfort of a mother and/or comfort of a baby.

The microphone may be protected from contact with milk, and this may be achieved in various ways. For example, in the example embodiment of Figure 2b, the microphone 230 is surrounded by the material of the nipple chamber wall 214 and therefore cannot come into contact with expressed milk or other fluid. In the example embodiments of Figures 3, 4 and 6, the microphone 330, 430, 630 is positioned on the external side of the interior wall(s) of the nipple chamber, therefore does not come into contact with expressed milk during normal use of the nipple shield 201. In the example embodiment of Figure 5, the microphone 530 will come into contact with expressed milk and may be surrounded by fluid-resistant material (e.g. silicone, poly(p-xylylene) (Parylene) or PTFE (e.g. Teflon) coatings). Alternatively, the microphone 530 may be made from a fluid-resistant material which does not require protection from contact with fluid/milk.

In some example embodiments, the microphone 230 is selected from microphone types which are able to withstand high temperatures (e.g. 100 °C). This is in order to allow the nipple shield 201 to be sterilized before and/or after use (e.g. using boiling water). In the example embodiments of Figures 3-6, the microphone may or may not be removable from the nipple shield 201. In example embodiments where the microphone is removable from the nipple shield, use of temperature resistant materials is not necessary. Use of temperature resistant materials is also not necessary if the nipple shield 201 is to be sterilized using a method that does not require high temperatures, for example using chemical solutions.

In the example embodiments of Figures 2a-6, only one microphone is shown. In other example embodiments, the apparatus 200 may comprise more than one microphone. In some example embodiments, the acoustic signals from the multiple microphones may be averaged or otherwise linearly combined, then treated in the same way as a signal from a single microphone. In other example embodiments, the multiple signals may be used in combination in order to distinguish, for example, the periodic beat of vortex shedding from the background noise, for example by calculating a cross-correlation between the multiple signals.

In the example embodiments shown in Figures 2a-6, the nipple chamber 210 has a substantially cylindrical shape and comprises a single radial wall 214. In other example embodiments, the nipple chamber 210 may instead have other shapes (e.g. an oval prism or a polygonal prism) and/or may comprise more than one wall between the first chamber end 211 and the second chamber end 212 (e.g. for polygonal prism shapes). In some example embodiments, the shape formed by the interior surface(s) 517 of the nipple chamber 210 may be different to the shape formed by the exterior surface(s) 416 of the nipple chamber 210. The shape(s) of the nipple chamber 210 may be chosen based on one or more of: the comfort of the mother, the comfort of the baby, the ease of latching on for the baby, and the amount of turbulence produced in the milk flow for a particular shape.

As previously mentioned, the second chamber end 212 is substantially closed with at least one aperture 213 formed therein to allow the flow of expressed milk out of the nipple chamber 210 through the at least one aperture 213. 'Substantially closed with at least one aperture therein' should be understood to mean that the majority of the second end 212 of the nipple chamber 210 is formed of a wall (therefore, in contrast to the open first chamber end 211, a nipple cannot be received in the nipple chamber 210 through the second chamber end 212) but that this wall has at least one aperture 213 therein which allows milk to pass through the second chamber end 212. Figure 2a shows a dotted line demarcating the second chamber end 212 of nipple chamber 210 in one example embodiment, as well as aperture 213.

'Substantially closed with at least one aperture therein' may be parameterized using the relative areas of the second chamber end and the one or more apertures therein. Defining 'the total surface area of the second chamber end' as the total surface area that the wall of the second chamber end 212 would have if there were no apertures 213 therein, and 'the cross-sectional area of an aperture' to be the surface area of the region of wall that is missing due to the presence of the aperture 213, the term 'substantially closed' should be understood to mean that the sum of the respective cross-sectional areas (i.e. the cumulative cross-sectional area) of all of the apertures in the second chamber end is a small proportion of the total surface area of the second chamber end. For example, the cumulative cross-sectional area of the one or more apertures may be one or more of up to 5 %, 10 %, 15 % and 20 % of the total surface area of the second chamber end.

It will be appreciated that in some embodiments, all of the apertures in the second chamber end are suitable for milk to pass through and for causing turbulence. In other embodiments, in addition to one or more apertures for milk flow there may be included one or more apertures which milk does not pass through (e.g. which are sized such that milk cannot pass through) and/or which do not cause turbulence (such as apertures for breathing/air circulation). It will be appreciated that in these embodiments, the sum of the respective cross-sectional areas for all of the apertures (i.e. both turbulence-causing and non-turbulence-causing apertures) is a small proportion (e.g. less than at least one of up to 5 %, 10 %, 15 %, 20 %) of the total surface area of the second chamber end.

The one or more apertures 213 have the effect of causing turbulence in the milk flow, and the number, size, shape and/or position of the one or more apertures 213 may be chosen to influence this effect. In the example embodiment of Figure 2, the at least one aperture 213 is circular in shape, but in other example embodiments the at least one aperture 213 may be another shape (e.g. oval or polygonal). When chosen for use with a human mammary gland, the at least one aperture 213 may be sized between 0.5 mm and 5 mm in an appropriate dimension. The appropriate dimension to consider the size of will differ for different shapes of apertures, e.g. the diameter of a circular aperture, a major or minor diameter of an oval, or the side length of a regular polygon. In example embodiments with two or more apertures, different apertures can have different shape and/or sizes, e.g. one larger circular aperture can be surrounded by five smaller circular apertures. The number, shapes, sizes and/or position of the one or more apertures may be chosen based on one or more of: the amount of turbulence produced in the milk flow, the pattern of turbulence produced in the milk flow, the comfort for mother/baby, the ease of milk flow produced, or any other consideration, and it will be appreciated that the amount of turbulence produced will vary depending on the number, shapes, sizes and/or positions of the one or more apertures. The relation between the cross-sectional area of the one or more apertures and the total surface area of the second chamber end may also be chosen to influence the amount and/or pattern of turbulence that is caused in the milk flow. For example, if the apertures form a small part (e.g. 10%) of the second chamber end, there may more turbulence produced than if the apertures form a larger part (e.g. 50%).

In the example embodiments of Figures 2a-6, the nipple shield 210 may be made from silicone. Silicone may be chosen because it is strong, soft, thin, flexible, cheap, impermeable and temperature resistant. In these and other example embodiments, other materials may be used.

The base portion 220 of the apparatus 200 may be of any size, e.g. to maximize comfort and practicality for the mother. In some example embodiments, the apparatus 200 may not include a base portion 220. Instead, the nipple chamber 210 and microphone 230 may be otherwise attached or held to the nipple of the mammary gland when in use.

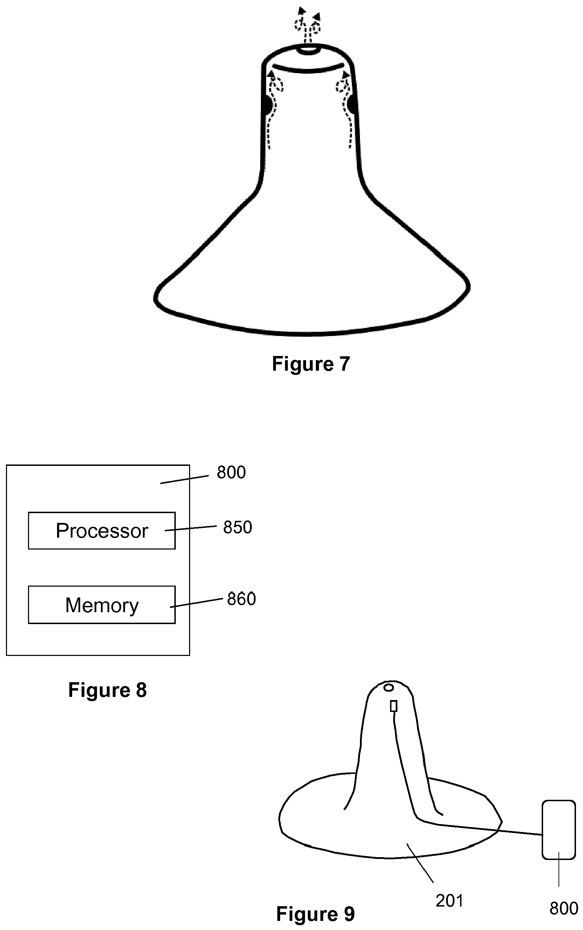

When in use, the apparatus 200 is positioned over a nipple and the mother expresses milk which flows through the nipple chamber 210 and out of the one or more apertures 213 in the second chamber end 212 (e.g. into a baby's mouth). In the example embodiments of the present disclosure, the milk flow through the nipple chamber 210 is at least partially turbulent, allowing the microphone 230 to detect an acoustic signal produced by the turbulent milk flow. Turbulence in the milk flow is caused by the flow through a constriction or past an obstruction in the milk flow (as shown in Figure 7), and vortices may form (and be shed) at or near at least some of the constrictions and/or obstructions. The turbulence in the milk flow creates pressure fluctuations which can be detected as an acoustic signal by microphone 230. This acoustic signal can be used to determine a flow speed of milk through the one or more apertures 213.

The aperture(s) 213 in the second chamber end 212 is one example of a constriction which causes turbulence in the milk flow. An example of an obstruction in the milk flow is one or more structures on the interior surface(s) of the nipple chamber (as shown in Figure 7). In some example embodiments, these one or more structures are physical protrusions from the interior surface. In other example embodiments, regions of the interior surface 517 can be formed from (or coated with) hydrophilic material or hydrophobic material to influence the fluid flow towards or away from these regions. Combinations of (e.g. alternating regions) hydrophilic and hydrophobic regions could also be used to promote turbulence in the milk flow. Another example of an obstruction in the milk flow is one or more concavities (or equivalently 'depressions') in the interior surface(s) of the nipple chamber 210. This could be considered as a 'negative obstruction'.

Turbulence can also be caused and/or influenced by other features of the nipple chamber 210, and the nipple chamber 210 may be specifically designed to increase the amount of turbulence in the milk flow, producing a larger and more easily detectable acoustic signal. For example, the dimensions of the nipple chamber 210 can influence the fluid flow and turbulence therein. The nipple chamber 210 is longitudinally dimensioned so that the nipple-end of the received nipple is held proximal to the second chamber end 212 (if the nipple-end is not held sufficiently proximal to the second chamber end 212, the milk will flow over a large distance between being expressed and passing through the one or more apertures 213 and the flow may become insufficiently turbulent/too laminar for suitable pressure fluctuations to be produced or for steady vortex shedding to occur.)

In some example embodiments, the distance between a received nipple-end and the second chamber end 212 may be less than one or more of 10 mm, 9 mm, 8 mm, 7 mm, 6 mm, 5 mm, 4 mm, 3 mm, 2 mm and 1 mm, and/or the nipple chamber 210 may have a longitudinal dimension of 15-50 mm. It will be understood that the nipple chamber 210 may be longitudinally dimensioned to achieve this for a typical range of nipple sizes for a population, e.g. an adult female human population. Separate apparatus could be manufactured for different classes of nipple size - for example, the adult female human population could be classified into 'small, 'medium' and 'large' average nipple sizes, and separate 'small', 'medium' and 'large' apparatus could be manufactured such that the distance between a received nipple-end and the second chamber end 212 is less than one or more of 10 mm, 9 mm, 8 mm, 7 mm, 6 mm, 5 mm, 4 mm, 3 mm, 2 mm and 1 mm for a respective 'small', 'medium' and 'large' received nipple of the average size for the respective class. It will be appreciated that this feature should not be interpreted as limited to considering 'the distance between the (received) nipple-end and the second chamber end 212, whilst the apparatus is in use on a mammary gland'. Instead, this feature encompasses an apparatus with a nipple chamber 210 which has these dimensions relative to an average nipple size.

The nipple chamber 210 may also be radially dimensioned so that a wall (e.g. the radial wall of a cylindrical nipple chamber) is sufficiently close to a received nipple to enhance turbulence and/or to prevent an insufficiently turbulent flow occurring if the nipple chamber 210 is substantially wider than the received nipple. In some example embodiments, the wall may be within a proximity of at least one of 5 mm, 4 mm, 3 mm, 2 mm and 1 mm of the received nipple, and/or the nipple chamber may have a radial dimension of 7-25 mm. As above, the nipple chamber 210 may be sized to achieve this for a typical range of nipple sizes for a population, e.g. an adult female human population, and separate apparatus could be manufactured for different classes of nipple size, i.e. such that a wall of the nipple chamber 210 is within a proximity of at least one of 5 mm, 4 mm, 3 mm, 2 mm and 1 mm of a respective 'small', 'medium' and 'large' received nipple of the average size for the respective class. As above, it will be appreciated that this feature should not be interpreted as limited to 'within a proximity of 5 mm, 4mm, 3 mm, 2mm and 1 mm of the received nipple whilst the apparatus is in use on a mammary gland'. Instead, this feature encompasses an apparatus with a nipple chamber 210 which has these dimensions relative to an average nipple size.

The relative longitudinal and radial dimensions of the nipple chamber can also influence the amount of turbulence caused. For example, for a given radial dimension, a shorter longitudinal dimension may produce more turbulence.

In the example embodiments of the present disclosure, it is important that the milk flow through the nipple chamber 210 is at least partially turbulent to allow for the microphone to detect an acoustic signal produced by turbulent milk flow. However, it will be appreciated that not all possible milk flows through all possible nipple chambers will (necessarily) be turbulent. Particular configurations of apparatus 200 could be specifically designed or chosen to ensure that milk flow through the nipple chamber 210 is turbulent, such as by considering whether the Reynolds number for a considered particular configuration is above the turbulence threshold.

In some example embodiments, apparatus 200 can be designed so that the turbulent fluid flow can be characterized by the dimensionless Strouhal number. In this case, the Strouhal number $St$ can be used to determine the flow speed of the turbulent flow of milk through apparatus 200. The Strouhal number relates the frequency $f$ of vortex shedding in the flow, a characteristic length $L$ for the flow and the characteristic flow speed $U$, according to:

$$St = \frac{fL}{U}.$$

This can be rearranged to give:

$$U = \frac{fL}{St} = f \ \frac{L}{St}.$$

That is, the flow speed $U$ is directly proportional to the frequency $f$ of vortex shedding in the flow, scaled by an $L/St$ factor. For fluid flow through a nipple shield 201, the characteristic flow speed U is the flow speed away from the one or more apertures and the characteristic length $L$ is a dimension (e.g. a diameter) of the one or more apertures 213. As characteristic length $L$ is selected to represent the particular apparatus configuration and the Strouhal number is known to be roughly constant for different turbulent fluid flows in a particular apparatus configuration, the speed of fluid flow through a particular apparatus configuration is proportional to the frequency of vortex shedding of the turbulent flow, scaled by a constant $L/St$ factor.

This relationship is used in at least some example embodiments to determine the speed of milk flow through the one or more apertures 213. For a particular configuration of apparatus 200, characteristic length $L$ and Strouhal number $St$ can be determined, i.e. a calibration factor ($L/St$) can be determined for the apparatus. Frequency of vortex shedding can then be determined (as discussed in the next paragraph) for a particular flow (e.g. in a particular breast feeding session), giving the flow speed for expressed milk passing through the nipple chamber 201 according to $U = f \times L/St$. Multiplying the calculated flow speed $U$ by the cross-sectional area of the one or more apertures 213 gives the volumetric flow rate of milk flowing through and out of the nipple chamber 210.

Integrating the volumetric flow rate with respect to time over the expressing session gives the volume of milk expressed by the mammary gland in the expressing session (thus allowing the volume of milk consumed by a baby in a feeding session of known duration to be determined.)

The frequency of vortex shedding is determined using an acoustic signal detected by the microphone 230 of the nipple shield 201. In some example embodiments, microphone 230 may be a contact microphone which senses audio vibrations through contact with solid objects, in the present case the wall(s) 214 of the nipple chamber 210. Audio vibrations of vortex shedding in the milk are transmitted from the milk to the walls 214 of the nipple chamber 210 to the contact microphone. In other example embodiments, other types of microphones (including pressure sensors suitable for sensing vibrations) could be used. The periodic shedding of vortices at the apertures 213 produces a detectable periodic acoustic signal which is detected by the microphone 230 and which is distinguishable from other background noise due to its periodic nature. In some example embodiments, multiple acoustic signals (e.g. from multiple microphones or from a single microphone 230 at different time periods within a feeding session) can be used in combination to distinguish the periodic signal from the (aperiodic) background noise (e.g. by calculating a cross-correlation between the multiple signals).

In other example embodiments, the acoustic signal produced by turbulent milk flow could be used to determine flow speed using other methods, without using the Strouhal number or considering vortex shedding. For example, the amplitude of the acoustic signal could be considered.

The detected acoustic signal is received by an apparatus 800 as shown in Figure 8. Apparatus 800 comprises a processor 850 and a memory 860 including computer program code, and the memory 860 and computer program code are configured to, with processor 850, cause the apparatus 800 to receive an acoustic signal from a microphone 230 located proximal to the nipple chamber 210 of a second apparatus 200, wherein the nipple chamber has opposing first 211 and second 212 chamber ends, is configured to receive a nipple of a mammary gland through the first chamber end 211 and is dimensioned to hold the nipple-end of a received nipple proximal to the opposing second chamber end 212, the second chamber end 212 being substantially closed with one or more apertures 213 therein to allow milk to flow from the received nipple out of the second chamber end 212 and which produce turbulence in the milk flow, wherein the received acoustic signal is from the turbulent milk flow; and to determine the speed of milk flow through the one or more apertures 213 using the acoustic signal.

The apparatus 800 may be further caused to determine a volumetric flow rate or volume of milk expressed by the mammary gland by multiplying the speed of milk flow by the cross-sectional area of the one or more apertures 213 and, for the volume, by integrating the volumetric flow rate with respect to time over the expressing session.

As apparatus 200 detects an acoustic signal produced by turbulence in the milk flow through the nipple chamber 210 and apparatus 800 receives an acoustic signal and determines the speed of milk flow through the one or more apertures 213 using the received acoustic signal, apparatus 200 and 800 are configured to be used together to determine a speed of expressed milk passing through the one or more apertures of the nipple chamber 210.

As discussed above, in some example embodiments apparatus 800 may be caused to determine the frequency of vortex shedding from the acoustic signal, and to determine the speed of milk flow using the determined signal.

The apparatus 800 may determine the speed of milk flow by explicitly using the Strouhal number and the characteristic length for flow through a particular configuration of nipple chamber or by using a calibration factor (which was calculated from the Strouhal number and characteristic length).

Also as discussed above, in other example embodiments, the acoustic signal produced by turbulent milk flow could be used by the apparatus 800 to determine flow speed using other methods, without using the Strouhal number or considering vortex shedding. For example, the average sound level of the acoustic signal may be correlated with the milk flow speed (i.e. a faster flow speed may produce a correspondingly louder acoustic signal) and the apparatus 800 could therefore consider the average amplitude of the acoustic signal to determine the flow speed.

In some example embodiments, the apparatus 800 may be a standalone device for determining milk flow speed (and potentially other parameters of the milk or milk flow).

In other example embodiments, the apparatus 800 may be a smartphone running an application which can receive the acoustic signal and determine the milk flow speed therefrom.

In some example embodiments, the apparatus 800 and the nipple shield apparatus 201 of Figures 2a-6 may be physically connected or connectable (as shown in Figure 9) to allow transmission of the acoustic signal from the microphone to the apparatus 800. In other example embodiments, the nipple shield apparatus 201 may comprise a transmitter which can transmit the detected acoustic signal from apparatus 201 to apparatus 800 without requiring a physical connection to be formed.

In yet further example embodiments, the apparatus 800 may form part of the nipple shield apparatus 201, i.e. the processor 850 and memory 860 may be comprised as part of the nipple shield apparatus. In these further example embodiments, the nipple shield apparatus 201 may comprise an interface for displaying the determined flow speed (and/or volumetric flow rate and/or volume), and/or the determined flow speed (and/or volumetric flow rate and/or volume) may be transmitted (via a wired or wireless connection) to another apparatus. In some example embodiments, the apparatus 800 may be permanently connected to the microphone 230 which is comprised by the nipple shield 201 but which is removable from the nipple chamber (e.g. such as in the example embodiments of Figures 3, 4 and 6).

Each of these different example embodiments may provide advantages in various use cases. For example, by using a smartphone device running an application, a mother would not have to purchase and carry around a separate device in order to measure the amount of breast milk that she expresses/her baby consumes. On the other hand, a standalone device may have a relatively long battery life, meaning that the mother would only need to recharge the device relatively infrequently. A mother and baby clinic (or other locations where a large number of mothers may use the apparatus 800) might choose to provide a standalone device so that it could be easily shared between multiple users.

As another example, embodiments with a wireless connection between apparatus 800 and apparatus 201 might be more convenient and less cumbersome for a user than embodiments with a wired connection. On the other hand, embodiments with a wired connection might be easier to repair if problems arise with the connection than those with a wireless connection.

As another example, when the two apparatus 201, 800 are combined into a single apparatus, a mother only has to carry that single apparatus which is potentially more convenient than carrying separate apparatus 201 and apparatus 800. On the other hand, when apparatus 201 and 800 are separate, the processor/memory parts of the apparatus 800 do not need to be protected when the nipple shield part of apparatus 201 is being cleaned/sterilized.

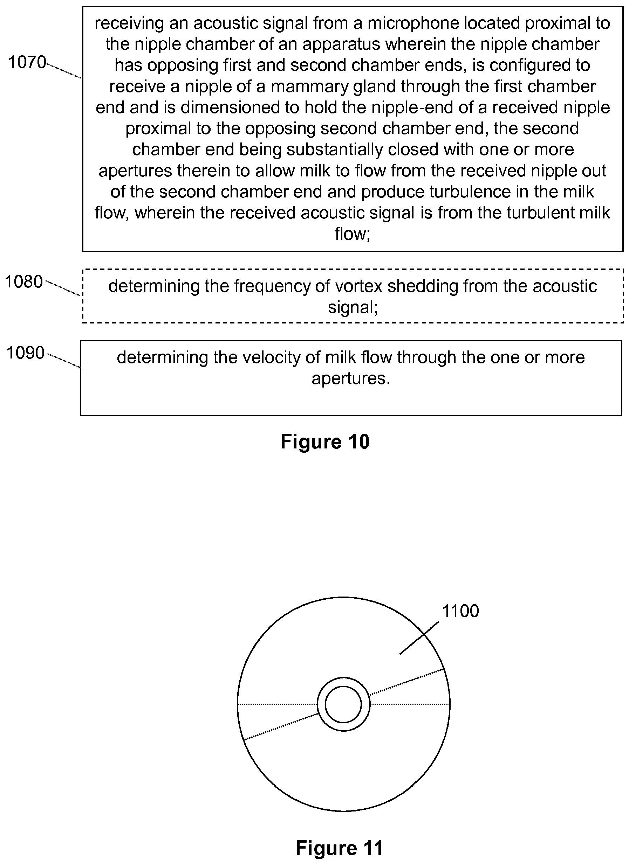

Figure 10 illustrates the steps 1070 and 1090 of a method as described herein and which can be performed using the apparatus 800. The method generally comprises: receiving an acoustic signal from a microphone located proximal to the nipple chamber of an apparatus wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk to flow from the received nipple out of the second chamber end and which produce turbulence in the milk flow, wherein the received acoustic signal is from the turbulent milk flow 1070; and determining the speed of milk flow through the one or more apertures 1090.

As discussed above, in some example embodiments the acoustic signal received in step 1070 may be produced by the shedding of vortices in the turbulent milk flow, and the method may further comprise determining the frequency of vortex shedding from the acoustic signal 1080 (as shown in Figure 10). The speed of milk flow may be determined (in step 1090) using this frequency of vortex shedding.

It will be appreciated that one or more of the previously mentioned dimensional properties of the apparatus (including the shape of the nipple chamber, the radial dimension of the nipple chamber, the longitudinal dimension of the nipple chamber, the relative longitudinal and radial dimensions of the nipple chamber, the distance between one or more of the walls of the nipple chamber and the received nipple, the distance between the second chamber-end and the received nipple-end, the shape of the one or more apertures in the second chamber end, the size of an appropriate dimension of the one or more apertures in the second chamber end, the cumulative surface cross-sectional area of the one or more apertures as a proportion of the total surface area of the second chamber end of the nipple chamber, and one or more structures on an interior wall of the nipple chamber) can be chosen in combination to provide turbulence in the milk flow.

Figure 11 illustrates schematically a computer/processor readable medium 1100 providing a program configured to perform, control or enable one or more of the method steps 1070, 1090 of Figure 10. In this example, the computer/processor readable medium is a disc such as a Digital Versatile Disc (DVD) or a Compact Disc (CD). The computer program code may be distributed between the multiple memories of the same type, or multiple memories of a different type, such as ROM, RAM, flash, hard disk, solid state, etc.

As previously mentioned, breast feeding is known to have numerous benefits for both mother and baby which infant formula or other alternatives lack. However, sometimes the amount of breastmilk consumed by a baby may be insufficient to support normal growth and development. For example, the mother may have difficulty expressing a sufficient quantity of milk. Alternatively, the baby may have difficulty latching onto the mother's nipple. It is therefore important to monitor how much milk is consumed by the baby to ensure that the baby receives sufficient nutrients for growth and development. Similar considerations may apply to both human and non-human mammals. Example embodiments of the invention may be used for livestock for example.

By virtue of a microphone being located proximally to a nipple chamber through which milk can flow, example embodiments of the present disclosure provide various technical effects. For example, the use of a microphone as described in the present disclosure allows the amount of milk consumed by a baby in a feeding session to be determined. Some example embodiments may allow this to be determined/monitored in real-time, i.e. during a feeding session rather than after a feeding session. Use of a microphone as described in the present disclose may also allow the amount of milk consumed to be determined with minimal disruption, inconvenience or discomfort to mother and baby, and without adversely affecting the milk flow or milk uptake by the baby.

Other example embodiments depicted in the figures have been provided with reference numerals that correspond to similar features of earlier described example embodiments. For example, feature number 230 can also correspond to numbers 330, 430 etc. These numbered features may appear in the figures but may not have been directly referred to within the description of these particular example embodiments. These have still been provided in the figures to aid understanding of the further example embodiments, particularly in relation to the features of similar earlier described example embodiments.

It will be appreciated to the skilled reader that any mentioned apparatus/device and/or other features of particular mentioned apparatus/device may be provided by apparatus arranged such that they become configured to carry out the desired operations only when enabled, e.g. switched on, or the like. In such cases, they may not necessarily have the appropriate software loaded into the active memory in the non-enabled (e.g. switched off state) and only load the appropriate software in the enabled (e.g. on state). The apparatus may comprise hardware circuitry and/or firmware. The apparatus may comprise software loaded onto memory. Such software/computer programs may be recorded on the same memory/processor/functional units and/or on one or more memories/processors/functional units.

In some example embodiments, a particular mentioned apparatus/device may be preprogrammed with the appropriate software to carry out desired operations, and wherein the appropriate software can be enabled for use by a user downloading a "key", for example, to unlock/enable the software and its associated functionality. Advantages associated with such example embodiments can include a reduced requirement to download data when further functionality is required for a device, and this can be useful in examples where a device is perceived to have sufficient capacity to store such preprogrammed software for functionality that may not be enabled by a user.

It will be appreciated that any mentioned apparatus/circuitry/elements/processor may have other functions in addition to the mentioned functions, and that these functions may be performed by the same apparatus/circuitry/elements/processor. One or more disclosed aspects may encompass the electronic distribution of associated computer programs and computer programs (which may be source/transport encoded) recorded on an appropriate carrier (e.g. memory, signal).

It will be appreciated that any "computer" described herein can comprise a collection of one or more individual processors/processing elements that may or may not be located on the same circuit board, or the same region/position of a circuit board or even the same device. In some example embodiments one or more of any mentioned processors may be distributed over a plurality of devices. The same or different processor/processing elements may perform one or more functions described herein.

With reference to any discussion of any mentioned computer and/or processor and memory (e.g. including ROM, CD-ROM etc), these may comprise a computer processor, Application Specific Integrated Circuit (ASIC), Field-Programmable Gate Array (FPGA), and/or other hardware components that have been programmed in such a way to carry out the inventive function.

The applicant hereby discloses in isolation each individual feature described herein and any combination of two or more such features, to the extent that such features or combinations are capable of being carried out based on the present specification as a whole, in the light of the common general knowledge of a person skilled in the art, irrespective of whether such features or combinations of features solve any problems disclosed herein, and without limitation to the scope of the claims. The applicant indicates that the disclosed aspects/example embodiments may consist of any such individual feature or combination of features. In view of the foregoing description it will be evident to a person skilled in the art that various modifications may be made within the scope of the disclosure.

While there have been shown and described and pointed out fundamental novel features as applied to different example embodiments thereof, it will be understood that various omissions and substitutions and changes in the form and details of the devices described may be made by those skilled in the art without departing from the spirit of the invention. For example, it is expressly intended that all combinations of those elements which perform substantially the same function in substantially the same way to achieve the same results are within the scope of the invention. Moreover, it should be recognized that structures and/or elements shown and/or described in connection with any disclosed form or example embodiment may be incorporated in any other disclosed or described or suggested form or example embodiment as a general matter of design choice. Furthermore, in the claims means-plus-function clauses are intended to cover the structures described herein as performing the recited function and not only structural equivalents, but also equivalent structures. Thus although a nail and a screw may not be structural equivalents in that a nail employs a cylindrical surface to secure wooden parts together, whereas a screw employs a helical surface, in the environment of fastening wooden parts, a nail and a screw may be equivalent structures.

## Claims

1. An apparatus comprising:

    a nipple chamber having opposing first and second chamber ends, the chamber configured to receive a nipple of a mammary gland though the first chamber end and dimensioned to hold the nipple-end of a received nipple proximal to the opposing second chamber end, the second chamber end being substantially closed with one or more apertures therein to allow milk from the received nipple to flow out of the second chamber end and to produce turbulence in the milk flow; and
    a microphone located proximal to the nipple chamber and configured to receive an acoustic signal produced by the turbulent milk flow in the nipple chamber.

2. The apparatus of claim 1, wherein one or more of:

    one or more of the walls, or the radial wall, of the nipple chamber are within a proximity of at least one of 5mm, 4mm, 3mm, 2mm and 1 mm of the received nipple;
    the distance between the nipple-end and the second chamber end is less than one or more of: 10mm, 9mm, 8mm, 7mm, 6mm, 5mm, 4mm, 3mm, 2mm, 1mm.

3. The apparatus of claim 1, wherein one or more of:

    the cumulative cross-sectional area of the one or more apertures is one or more of up to 5 %, 10 %, 15 % and 20 % of the total surface area of the second chamber end of the nipple chamber;
    the size of an appropriate dimension of the one or more apertures in the second chamber end is in the range of 0.5mm to 5mm; and
    the shape of the one or more apertures in the second chamber end is circular, oval or polygonal.

4. The apparatus of claim 1, further comprising one or more structures on an interior wall of the nipple chamber, the one or more structures configured to increase turbulence in the milk flow.

5. The apparatus of claim 3, wherein the structures comprise one or more of: protrusions, concavities, hydrophilic regions and hydrophobic regions.

6. The apparatus of claim 1, further comprising:

    at least one processor; and
    at least one memory including computer program code,

    the at least one memory and the computer program code configured to, with the at least one processor, cause the apparatus to perform at least the following:

        measure the acoustic signal produced by the turbulent milk flow in the nipple chamber; and determine the speed of milk flow through the one or more apertures using the measured acoustic signal.

7. The apparatus of claim 6, wherein the apparatus is configured to determine the speed of milk flow through the one or more apertures using the measured acoustic signal by:

        determining, from the acoustic signal, the frequency of vortex shedding in the turbulent milk flow; and
        determine the speed using the determined frequency.

8. The apparatus of claim 1, wherein the mammary gland is a human mammary gland.

9. The apparatus of claim 1, wherein the microphone is at least one of:

        embedded within a wall of the nipple chamber such that the surface roughness of the interior surface of the wall in the region where the microphone is embedded is within 5% of the surface roughness of the interior surface of the wall in the regions where the microphone is not embedded;
        removably located in a microphone cavity in a wall of the nipple chamber, the microphone cavity positioned and dimensioned such that the surface roughness of the interior surface of the wall in the region of the microphone cavity is within 5% of the surface roughness of the interior surface of the wall in the regions away from the microphone cavity;
        located on an exterior or interior wall surface of the nipple chamber; or
        located at an opening of a cavity within a wall of the nipple chamber, wherein the opening is located at a wall surface of the nipple chamber and the cavity is shaped and dimensioned so that it provides an acoustic impedance match with the microphone.

10. An apparatus comprising:

        at least one processor; and

at least one memory including computer program code,

the at least one memory and the computer program code configured to, with the at least one processor, cause the apparatus to perform at least the following:

receive an acoustic signal from a microphone located proximal to the nipple chamber of a second apparatus, wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk from the received nipple to flow out of the second chamber end and produce turbulence in the milk flow, wherein the received acoustic signal is produced by the turbulent milk flow; and

determine the speed of milk flow through the one or more apertures using the received acoustic signal.

11. The apparatus of claim 10, wherein the microphone is located proximal to the nipple chamber of the second apparatus by being comprised by the second apparatus.

12. The apparatus of claim 11, wherein the computer program code is further configured to cause the apparatus to:

determine, from the acoustic signal, the frequency of vortex shedding in the turbulent milk flow; and

determine the speed of milk flow through the one or more apertures using the determined frequency.

13. The apparatus of claim 12, wherein the computer program code is further configured to cause the apparatus to determine the speed of milk flow using the determined frequency, the Strouhal number for milk flow through the nipple chamber and a characteristic length of the nipple chamber.

14. A method comprising:

receiving an acoustic signal from a microphone located proximal to the nipple chamber of an apparatus wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk from the received nipple to flow out of the second chamber end and produce turbulence in the milk flow, wherein the received acoustic signal is produced by the turbulent milk flow; and

determining the speed of milk flow through the one or more apertures using the received acoustic signal.

15. A computer program configured to cause an apparatus to:

receive an acoustic signal from a microphone located proximal to the nipple chamber of a second apparatus, wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk from the received nipple to flow out of the second chamber end and produce turbulence in the milk flow, wherein the received acoustic signal is produced by the turbulent milk flow; and

determine the speed of milk flow through the one or more apertures using the received acoustic signal.

Figure 1

Figure 2a

Figure 2b

**Figure 3**

**Figure 4**

**Figure 5**

**Figure 6**

**Figure 7**

800

| Processor | — 850 |
|---|---|

| Memory | — 860 |
|---|---|

**Figure 8**

201          800

**Figure 9**

1070 receiving an acoustic signal from a microphone located proximal to the nipple chamber of an apparatus wherein the nipple chamber has opposing first and second chamber ends, is configured to receive a nipple of a mammary gland through the first chamber end and is dimensioned to hold the nipple-end of a received nipple proximal to the opposing second chamber end, the second chamber end being substantially closed with one or more apertures therein to allow milk to flow from the received nipple out of the second chamber end and produce turbulence in the milk flow, wherein the received acoustic signal is from the turbulent milk flow;

1080 determining the frequency of vortex shedding from the acoustic signal;

1090 determining the velocity of milk flow through the one or more apertures.

**Figure 10**

1100

**Figure 11**

Europäisches
Patentamt
European
Patent Office
Office européen
des brevets

# EUROPEAN SEARCH REPORT

Application Number

EP 17 17 2532

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | US 2008/167579 A1 (EZRA LIOR BEN [IL] ET AL) 10 July 2008 (2008-07-10) * paragraphs [0002], [0022]; figures 1,2,17 * ----- | 1-15 | INV. G01F1/66 A61B5/00 ADD. G01F1/32 G01P5/10 |
| Y | US 2016/223376 A1 (DALY JOHN GERARD [IE] ET AL) 4 August 2016 (2016-08-04) * paragraphs [0001], [0004], [0058], [0087], [0089], [0121], [0020]; claim 93; figure 1 * ----- | 1-15 | |
| A | US 2006/011571 A1 (SILVER BRIAN H [US]) 19 January 2006 (2006-01-19) * paragraph [0083] * ----- | 3 | |

TECHNICAL FIELDS
SEARCHED      (IPC)

G01F
A61B
G01P
A61J

The present search report has been drawn up for all claims

| Place of search | Date of completion of the search | Examiner |
|---|---|---|
| The Hague | 10 November 2017 | Reeb, Bertrand |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 17 17 2532

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2017

| Patent document cited in search report | | | Publication date | Patent family member(s) | | | Publication date |
|---|---|---|---|---|---|---|---|
| US 2008167579 | A1 | | 10-07-2008 | CN | 1894578 | A | 10-01-2007 |
| | | | | EP | 1653906 | A2 | 10-05-2006 |
| | | | | US | 2008167579 | A1 | 10-07-2008 |
| | | | | WO | 2005016220 | A2 | 24-02-2005 |
| US 2016223376 | A1 | | 04-08-2016 | EP | 3047242 | A1 | 27-07-2016 |
| | | | | US | 2016223376 | A1 | 04-08-2016 |
| | | | | WO | 2015040607 | A1 | 26-03-2015 |
| US 2006011571 | A1 | | 19-01-2006 | AU | 2006266099 | A1 | 11-01-2007 |
| | | | | CA | 2613843 | A1 | 11-01-2007 |
| | | | | CN | 101528186 | A | 09-09-2009 |
| | | | | EP | 1906910 | A2 | 09-04-2008 |
| | | | | IL | 188328 | A | 30-04-2015 |
| | | | | JP | 4964879 | B2 | 04-07-2012 |
| | | | | JP | 2009504200 | A | 05-02-2009 |
| | | | | KR | 20080038132 | A | 02-05-2008 |
| | | | | US | 2006011571 | A1 | 19-01-2006 |
| | | | | US | 2011240586 | A1 | 06-10-2011 |
| | | | | WO | 2007005427 | A2 | 11-01-2007 |

EPO FORM P0459

For more details about this annex : see Official Journal of the European Patent Office, No. 12/82